# EUROPÄISCHE PATENTSCHRIFT

(11) **EP 4 106 825 B1**
(45) Veröffentlichungstag und Bekanntmachung des Hinweises auf die Patenterteilung: **10.09.2025**
(21) Anmeldenummer: 21706239.7
(22) Anmeldetag: 16.02.2021
(51) Int. Cl.: A61L 26/00, A61L 27/16, A61L 27/50, A61L 31/14, A61K 9/00, G01K 11/12

(54) **OPHTHALMOLOGISCHE ZUSAMMENSETZUNG UND DETEKTIONSVORRICHTUNG FÜR EIN MEDIZINISCHES GERÄT**
OPTHALMOLOGICAL COMPOSITION AND DETECTION APPARATUS FOR A MEDICAL DEVICE
COMPOSITION OPHTALMOLOGIQUE ET APPAREIL DE DÉTECTION POUR UN DISPOSITIF MÉDICAL

(30) Priorität: 20.02.2020 DE 102020104540
(43) Veröffentlichungstag der Anmeldung: 28.12.2022
(73) Patentinhaber: Carl Zeiss Meditec AG, 07745 Jena (DE)
(72) Erfinder: MASCH, Jennifer-Magdalena, 10317 Berlin (DE); THALLER, Michael, 10585 Berlin (DE)
(74) Vertreter: Hofstetter, Schurack & Partner
(86) Internationale Anmeldenummer: PCT/EP2021/053755
(87) Internationale Veröffentlichungsnummer: WO 2021/165251

(56) Entgegenhaltungen:
- EP-B1- 3 089 766
- WO-A1-2013/020917
- WO-A1-2016/108071
- WO-A1-2018/156659
- GUTIERREZ M. I ET AL: "Therapy ultrasound equipment characterization: Comparison of three techniques", 2001 CONFERENCE PROCEEDINGS OF THE 23RD ANNUAL INTERNATIONAL CONFERENCE OF THE IEEE ENGINEERING IN MEDICINE AND BIOLOGY SOCIETY, vol. 2008, 1 January 2008 (2008-01-01), IEEE PISCATAWAY, NJ, USA,, pages 5117 - 5120, XP055807266, ISSN: 1094-687X, DOI: 10.1109/IEMBS.2008.4650365
- DATABASE WPI Week 200528, Derwent World Patents Index; AN 2005-270197, XP002803073
- DATABASE WPI Week 201625, Derwent World Patents Index; AN 2016-13868E, XP002803074

## Beschreibung

### Technisches Gebiet

Die Erfindung betrifft eine ophthalmologische Zusammensetzung.

### Stand der Technik

Bei der Kataraktchirurgie werden während des ophthalmologischen Eingriffs klare und gelartige ophthalmologische Zusammensetzungen (sogenannte ophthalmic viscosurgical devices bzw. ophthalmic viscoelastic devices, OVDs) verwendet, um in der Vorderkammer des Auges Platz zu schaffen und zu erhalten und das Hornhautendothel zu schützen. Konventionelle OVDs bieten viskose und elastische Eigenschaften, je nach dem gewünschten Grad der Volumenerhaltung und des Beschichtungsschutzes. Insbesondere bei der Phakoemulsifikation bietet die Anwendung von OVDs erhebliche Vorteile, um das Risiko für Komplikationen zu verringern.

GUTIERREZ M. I ET AL ("Therapy ultrasound equipment characterization: Comparison of three techniques",2001 CONFERENCE PROCEEDINGS OF THE 23RD ANNUAL INTERNATIONAL CONFERENCE OF THE IEEE ENGINEERING IN MEDICINE AND BIOLOGY SOCIETY, Bd. 2008, 1. Januar 2008 (2008-01 -01), Seiten 5117-5120, XP055807266, IEEE PISCATAWAY, NJ, USA, ISSN: 1094-687X, DOI: 10.1109/IEMBS.2008.4650365) zeigt einen Vergleich verschiedener Ultraschalltechniken auf.

WO 2016/108071 A1 offenbart eine passive planare thermooptische Struktur zur Erkennung eines Bereichs mit veränderter Temperatur auf der Haut eines Patienten, die einen thermoaktiven Farbstoff enthält.

Aus Thomson Scientific (London, GB; AN 2005-270197 & KR 2004 0102473 A, KOREA ELECTRIC POWER CORP, 2004-12-08) ist eine Vorrichtung zur Verringerung von Hitzeschäden am Auge während einer Kataraktoperation durch Verwendung eines thermochromen Kunststoffs bekannt, der seine Farbe mit der Temperatur ändert.

WO 2013/020917 A1 offenbart eine opthalmologische Zusammensetzung, die eine wässrige Lösung von mindestens einem viskoelastischen Polysaccharid enthält, das kovalent an mindestens einen Farbstoff gebunden ist.

WO 2018/156659 A1 offenbart ein Verfahren zur Bereitstellung eines temperaturempfindlichen Elements, das ein chirurgisches Instrument umgibt, um einem Bediener des Instruments eine visuelle Anzeige der Spitzentemperatur zu geben.

EP 3 089 766 B1 offenbart eine ophthalmologische viskoelastische Vorrichtung (OVD) zur Verwendung in einem Verfahren zur Behandlung eines Auges einer Person, wobei die OVD ein Material umfasst, das seine Viskosität bei Anwendung von vordefinierten Stimuli ändert.

Eine große Umfrage in den Vereinigten Staaten und Kanada dokumentierte für derartige Augenoperationen eine Inzidenz von 0,037 % für kornealen Wundverbrennung (Sorensen Tet al. (2012): Ultrasound-induced corneal incision contracture survey in the United States and Canada. J Cataract Refract Surg, 38(2):227-233). Bei der kornealen Wundverbrennung (Hornhautverbrennungen) handelt es sich um eine relativ seltene, aber ernsthafte Komplikation bei der Phakoemulsifikation, deren Inzidenz gemäß Sorensen signifikant umgekehrt mit dem Operationsvolumen des Chirurgen assoziiert ist. Die Ultraschallbewegung einer zur Phakoemulsifikation verwendeten Phakoemulsifikationsnadel kann Wärme erzeugen, die bei einer bestimmten Inzisionstemperatur von etwa 60 °C oder höher zu einer akuten Kollagenschädigung an der Inzisionsstelle führen kann. Solche Verbrennungen sind häufig mit einem Hornhautödem und schwerem chirurgisch induziertem Astigmatismus verbunden. Darüber hinaus kann das Hornhautendothel durch einen Anstieg der Hornhauttemperatur irreversibel zerstört werden. Unter anderem tragen die Menge der eingesetzten Phakoenergie und das Fehlen einer ausreichenden Irrigation und Aspiration zur Wärmeerzeugung bei. Die Phakonadel wird daher in der Regel durch Irrigation gekühlt. Jede Störung des Flüssigkeitsstroms oder ein zu niedrig eingestellter Flüssigkeitsstrom kann jedoch zu einem unerwünschten Temperaturanstieg führen. Ein weiterer Risikofaktor für eine Hornhautverbrennung ist die Art des verwendeten OVDs. In Kombination mit Ultraschall können bestimmte OVDs exotherme Eigenschaften entwickeln, die zu einem zusätzlichen thermischen Energieeintrag führen.

### Darstellung der Erfindung

Aufgabe der vorliegenden Erfindung ist es, das Risiko einer kornealen Wundverbrennung während einer Augenoperation zu verringern.

Die Aufgabe wird erfindungsgemäß durch eine ophthalmologische Zusammensetzung gemäß Patentanspruch 1 gelöst. Vorteilhafte Ausgestaltungen mit zweckmäßigen Ausbildungen der Erfindung sind in den Unteransprüchen angegeben.

Ein erster Aspekt der Erfindung betrifft eine ophthalmologische Zusammensetzung gemäß Anspruch 1, welche wenigstens ein viskoelastisches Polymer umfasst. Erfindungsgemäß weist die ophthalmologische Zusammensetzung wenigstens eine thermoresponsive Verbindung auf, welche temperaturabhängig in einem vorbestimmten Wellenlängenbereich wenigstens eine physikalische Eigenschaft aus der Gruppe Farbe und Transmissionsgrad diskontinuierlich ändert, wobei die wenigstens eine thermoresponsive Verbindung in einem ersten Temperaturbereich unterhalb von 60 °C eine erste Farb- und/oder Transmissionseigenschaft und in einem oberhalb des ersten Temperaturbereichs liegenden zweiten Temperaturbereich eine von der ersten Farb- und/oder Transmissionseigenschaft unterschiedliche zweite Farb- und/oder Transmissionseigenschaft besitzt. Mit anderen Worten schlägt die Erfindung eine thermoresponsive ophthalmologische Zusammensetzung vor, welche beim Überschreiten einer bestimmten Temperaturschwelle thermochrom reagiert, das heißt ihre Lichtabsorptionscharakteristik im vorbestimmten Wellenlängenbereich diskontinuierlich verändert, und/oder thermotrop reagiert, das heißt ihre Transmission bzw. Lichtdurchlässigkeit im vorbestimmten Wellenlängenbereich aufgrund eines Phasenübergangs verändert. Die erfindungsgemäße ophthalmologische Zusammensetzung kann auch als OVD (opthalmologische viskoelastische Lösung) bezeichnet werden und generell unterschiedliche Viskositäten aufweisen. Beispielsweise kann die erfindungsgemäße OVD als dispersive OVD oder als kohäsive OVD oder als kombinierte dispersiv-kohäsive OVD ausgebildet sein. Obwohl Chirurgen bereits die Operationsparameter bei einer Phakoemulsifikation variieren, gibt es derzeit keine Rückmeldung über die Wirksamkeit dieser Parameter in Bezug auf die intraokulare Temperatur bzw. Temperaturverteilung. Mit Hilfe der erfindungsgemäßen OVD kann erstmals das Risiko einer kornealen Wundverbrennung während einer Augenoperation erheblich reduziert werden, da die OVD dem Chirurgen eine Rückmeldung über die intraokulare Temperatur und vor allem eine unmittelbare Warnung beim Überschreiten eines vorbestimmten Temperaturgrenzwerts gibt. Neben den konventionellen Merkmalen bereits bekannter OVDs stellt die zusätzliche thermoresponsive Eigenschaft der erfindungsgemäßen OVD einen Temperaturindikator dar, der es dem Chirurgen ermöglicht, die Operationstechnik bei Bedarf umgehend umzustellen, um Hitzeschäden zu vermeiden. Der Chirurg hat dadurch eine bessere Kontrolle darüber, wie sich sowohl die momentan angewandte Phakoenergie als auch die momentane Irrigations- und Aspirationsrate auf die intraokulare Temperatur auswirken. Diese Rückmeldung über die intraokulare Temperatur während der Phakoemulsifikation bei der Kataraktchirurgie macht die chirurgische Behandlung wesentlich sichererer, wobei die erfindungsgemäße ophthalmologische Zusammensetzung grundsätzlich natürlich nicht nur bei der Kataraktbehandlung, sondern auch im Rahmen anderer Operationstypen verwendet werden kann. Ein großer Vorteil wird für die Zugabe der thermoresponsiven Verbindung(en) zu dispersiven OVDs gesehen, die normalerweise während der Operation zum Schutz des nicht regenerativen Hornhautendothels eingesetzt werden. Ein intraoperatives chirurgisches Trauma kann ein irreversibles Endothel-Versagen verursachen. Da eine Wundheilung oder Regeneration des Hornhautendothels nicht stattfindet, bietet die dispersive OVD mit thermoresponsiven Eigenschaften sowohl einen Beschichtungsschutz des Hornhautendothels als auch einen Temperatursensor am Hornhautendothel. Damit liefert sie wichtige Informationen über Temperaturänderungen an dieser empfindlichen Zellschicht. Die thermoresponsive(n) Verbindung(en) innerhalb der OVD sind vorzugsweise so konzipiert, dass sie ihr Aussehen entweder von farblos zu farbig (Thermochromie) und/oder von transparent zu nicht transparent (Thermotropie) oberhalb einer Temperaturschwelle verändern. Zusätzlich zu einem Wechsel von farblos zu farbig oder von transparent zu nicht-transparent können auch mehrfache Farb- und/oder Transparenzveränderungen bei jeweils unterschiedlichen Temperaturgrenzwerten vorgesehen sein. Der Grad der Färbung/Transparenz der thermoresponsiven OVD bietet dem Chirurgen bei Erhöhung der intraokularen Temperatur die Möglichkeit, die intraokulare Temperatur anzuzeigen, mit geringer bis starker Färbung und/oder hoher bis geringer Transparenz. Vorzugsweise ist die thermoresponsive Verbindung nicht toxisch bzw. toxikologisch tolerierbar und/oder biologisch abbaubar.

In einer vorteilhaften Ausgestaltung der Erfindung ist vorgesehen, dass eine temperaturabhängige Änderung der wenigstens einen physikalischen Eigenschaft reversibel oder irreversibel ist. Im Fall einer reversiblen Änderbarkeit der thermoresponsiven Verbindung kann die ophthalmologische Zusammensetzung nicht nur das Überschreiten einer Grenztemperatur anzeigen, sondern auch das anschließende Unterschreiten, wenn beispielsweise ein Chirurg die Operationsparameter entsprechend korrigiert hat. Im Fall einer irreversiblen Änderbarkeit der thermoresponsiven Verbindung kann die ophthalmologische Zusammensetzung einen Abbruch der Operation signalisieren, beispielsweise bereits wenn ein unzulässig hoher Temperaturwert erreicht wurde. Alternativ oder zusätzlich ist es vorgesehen, dass die temperaturabhängige Änderung der wenigstens einen physikalischen Eigenschaft beim Überschreiten eines vorbestimmten Temperaturgrenzwerts innerhalb von höchstens 10 Sekunden, vorzugsweise innerhalb von höchstens 2 Sekunden, erfolgt. Mit anderen Worten ist es erfindungsgemäß vorgesehen, dass die thermoresponsive Verbindung ihre optische Eigenschaft möglichst schnell nach Überschreiten des Temperaturgrenzwerts diskontinuierlich ändert, nämlich vorzugsweise innerhalb von 10 s, 9 s, 8 s, 7 s, 6 s, 5 s, 4 s, 3 s, 2 s, 1 s oder weniger. Hierdurch wird eine möglichst zeitnahe Rückmeldung über einen unzulässigen Temperaturanstieg ermöglicht, wodurch eine entsprechend schnelle Korrektur der Operationsparameter vorgenommen werden kann, bevor es zu einer Schädigung von Augengewebe kommt.

Weitere Vorteile ergeben sich, indem die wenigstens eine thermoresponsive Verbindung ausgewählt ist aus einer Gruppe, die Polymere, interpenetrierende Polymernetzwerke, semiinterpenetrierende Polymernetzwerke, Flüssigkristalle, insbesondere cholesterische Flüssigkristalle, Pigmente, Farbstoffe, Tinten, Mikrokapseln und beliebige Kombinationen hieraus umfasst. Auf diese Weise können neben den thermoresponsiven Eigenschaften auch zusätzliche Eigenschaften der ophthalmologischen Zusammensetzung wie beispielsweise Rheologie, Mischbarkeit der Einzelkomponenten etc. optimal angepasst werden. Durch die Verwendung von Mikrokapseln ist es möglich, auch kurzkettige thermoresponsive Verbindungen und schlecht lösliche bzw. schlecht mit dem viskoelastischen Polymer mischbare thermoresponsive Verbindungen zu verwenden. Zur Mikroeinkapselung wird vorzugsweise eine farblose bzw. transparente Verbindung verwendet, in der die thermoresponsive(n) Verbindung(en) eingeschlossen wird bzw. werden. Beispielsweise können eine Gelatine, ein substituiertes oder unsubstituiertes (Meth)Acrylat, ein Glycosaminoglycan und dergleichen als Hülle der Mikrokapsel verwendet werden. In weiterer Ausgestaltung kann vorgesehen sein, dass die Mikrokapseln kovalent an das viskoelastische Polymer gebunden sind, wodurch ein besonders zuverlässiger Schutz gegen ein Herausdiffundieren der thermoresponsiven Verbindung(en) gewährleistet ist.

In einer weiteren vorteilhaften Ausgestaltung der Erfindung ist vorgesehen, dass die wenigstens eine thermoresponsive Verbindung wenigstens eine konstitutionelle Einheit umfasst, die ausgewählt ist aus einer Gruppe, die Poly(N-Isopropylacrylamid), Poly(N,N-Diethylacrylamid), Poly(methylvinylether), Poly(N-Vinylcaprolactam), ein Blockcopolymer aus Poly(ethylenoxid) und Poly(propylenoxid), ein Poly(pentapeptid) von Elastin, ein interpenetrierendes Netzwerk aus Polyacrylamid und Polyacrylsäure, und Copolymerisate hiervon umfasst. Unter einem Copolymerisat sind im Rahmen der vorliegenden Offenbarung nicht nur Copolymere mit zwei Monomertypen bzw. zwei Wiederholeinheiten, sondern auch Polymere mit drei Monomertypen bzw. Wiederholeinheiten (Triblock-Copolymere) oder vier oder mehr Monomertypen bzw. Wiederholeinheiten zu verstehen. Weiterhin umfasst der Begriff Copolymerisat im Rahmen der vorliegenden Offenbarung alle möglichen Abfolgen der konstitutionellen Wiederholeinheiten, also beispielsweise statistische Copolymere, alternierende Copolymere, Blockcopolymere, Pfropfcopolymere, Gradientcopolymere usw. Ebenso sind alle möglichen Taktizitäten, das heißt ataktische, isotaktische und syndiotaktische Anordnungen einzelner Wiederholeinheiten im Makromolekül als mitoffenbart anzusehen. Weiterhin können Polymerblends von unterschiedlichen Homopolymeren und/oder Copolymeren vorgesehen sein. Über die Auswahl der konstitutionellen Einheiten bzw. Wiederholeinheiten sowie über die Variation ihrer Zusammensetzung und der Art ihrer Verknüpfung können einerseits der Grenztemperaturwert für die Phasenänderung und andererseits die Farbe und die Transmissionseigenschaften oberhalb und unterhalb des Grenztemperaturwerts bzw. des Phasenübergangs optimal an den jeweiligen Einsatzzweck angepasst werden.

In einer weiteren vorteilhaften Ausgestaltung der Erfindung ist vorgesehen, dass das wenigstens eine viskoelastische Polymer ein Polysaccharid umfasst, das aus einer Gruppe ausgewählt ist, die Glykosaminoglykane, Cellulose, ein Celluloseether mit Methyl- und/oder Ethyl- und/oder Propylgruppen, insbesondere Hydroxypropylmethylcellulose, Hydroxyethylmethylcellulose und/oder Methylcellulose, ein Glycosaminoglykan, insbesondere Hyaluronsäure, Chondroitinsulfat, Dermatansulfat, Heparin, Heparansulfat, Keratansulfat, Alginsäure, Polymannuronsäure, Polyguluronsäure, Polyglucuronsäure, Amylose, Amylopektin, Callose, Chitosan, Polygalactomannan, Dextran, Xanthan und/oder eine beliebige Mischung hieraus, Copolymerisate hiervon und pharmakologisch akzeptable Salze hiervon umfasst. Hierdurch können insbesondere die viskoelastischen Eigenschaften des OVD optimal an den jeweiligen Nutzungszweck angepasst werden. Dabei kann grundsätzlich auch vorgesehen werden, dass das OVD zwei oder mehrere Polysaccharide des gleichen Typs umfasst, die sich nur hinsichtlich eines oder mehrerer Parameter unterscheiden können.

In einer weiteren vorteilhaften Ausgestaltung der Erfindung ist vorgesehen, dass die wenigstens eine thermoresponsive Verbindung, insbesondere über einen Spacer, kovalent mit dem wenigstens einen viskoelastischen Polymer verbunden ist. Hierdurch kann ein Herausdiffundieren der thermoresponsiven Verbindung aus der ophthalmologischen Zusammensetzung vorteilhaft vermieden werden. Gegebenenfalls kann ein Spacer bzw. ein Quervernetzer zur einfachen kovalenten Anbindung verwendet werden und/oder um etwaige sterische Hinderungen der thermoresponsiven Verbindung und/oder des viskoelastischen Polymers zu vermeiden. Alternativ oder zusätzlich ist es vorgesehen, dass die wenigstens eine thermoresponsive Verbindung in Form von Partikeln und/oder Mikrokugeln im wenigstens einen viskoelastischen Polymer verteilt vorliegt. Solche thermoresponsiven Partikel, beispielsweise Mikro- und/oder Nanopartikel, bzw. Mikrokugeln können beispielsweise durch die Einbindung von thermochromen Farbstoffen erreicht werden. Diese thermochromen Farbstoffe können reversibel stabile delokalisierte Elektronensysteme bilden, die in der Lage sind, Licht im visuellen Spektrum zu absorbieren, wenn die Temperatur steigt, beispielsweise durch reversible Ringschlussreaktionen. Alternativ oder zusätzlich ist es vorgesehen, dass das wenigstens eine viskoelastische Polymer und die wenigstens eine thermoresponsive Verbindung ein semiinterpenetrierendes und/oder interpenetrierendes Netzwerk bilden. Interpenetrierende Polymernetzwerke (IPN) zeichnen sich durch zwei oder mehrere Netzwerke aus, die zumindest teilweise auf molekularer Ebene verschränkt, aber nicht kovalent aneinander gebunden sind. Daher können sie nicht getrennt werden, es sei denn, dass chemische Bindungen gebrochen werden. Auch hierdurch kann ein Herausdiffundieren der thermoresponsiven Verbindung zuverlässig verhindert werden. Semiinterpenetrierende Polymernetzwerke (SIPN) zeichnen sich durch ein oder mehrere Netzwerke und ein oder mehrere lineare oder verzweigte Polymer(e) aus, die sich auf molekularer Ebene durchdringen. Damit können die linearen oder verzweigten Polymere prinzipiell von dem bzw. den konstituierenden Polymernetzwerk(en) getrennt werden, ohne chemische Bindungen zu brechen. Auch hier ergibt sich jedoch in der Regel ein zuverlässiger Schutz gegen ein unerwünschtes Herausdiffundieren.

Erfindungsgemäß wird die wenigstens eine thermoresponsive Verbindung derart ausgewählt, dass der für die Veränderung des Aussehens vorbestimmte Temperaturwert höchstens etwa 60 °C beträgt, da oberhalb dieser Temperatur mit Hornhautverbrennung gerechnet werden muss. Bevorzugt beträgt der vorbestimmte Temperaturwert höchstens 40 °C und liegt besonders bevorzugt im Temperaturbereich oberhalb der intraokularen Temperatur, das heißt zwischen etwa 35 °C und etwa 38 °C. Für den Fall, dass die thermoresponsive Verbindung ihre optische(n) Eigenschaft(en) reversibel ändern kann, würde die thermoresponsive Verbindung beim Unterschreiten eines Temperaturschwellenwerts dementsprechend wieder ihre ursprünglichen optischen Eigenschaften bzw. ihre Eigenschaften, die sie bei Raumtemperatur besitzt, annehmen. Der vorbestimmte Temperaturwert beim Erwärmen und der Temperaturschwellenwert beim Abkühlen können dabei in manchen Fällen identisch sein. Aufgrund von Hysterese-Effekten können sich in manchen Fällen beim Erwärmen und Wiederabkühlen auch unterschiedliche Schwellenwerte ergeben. Es kann vorgesehen sein, dass die wenigstens eine thermoresponsive Verbindung im ersten Temperaturbereich unterhalb von 60 °C, insbesondere unterhalb von 45°C und oberhalb von 38°C die erste Farb- und/oder Transmissionseigenschaft und im oberhalb des ersten Temperaturbereichs liegenden zweiten Temperaturbereich die von der ersten Farb- und/oder Transmissionseigenschaft unterschiedliche zweite Farb- und/oder Transmissionseigenschaft besitzt. Vorzugsweise besitzt die wenigstens eine thermoresponsive Verbindung nicht in beiden Zuständen eine sichtbare Lichtemission bzw. einen stark lichtstreuenden Zustand, da dies ein dauerhaft gefärbtes bzw. dauerhaft intransparentes oder zumindest nicht klar durchsichtiges OVD erzeugen würde, das die Sicht des Chirurgen während der Kataraktoperation beeinträchtigen könnte.

In einer weiteren vorteilhaften Ausgestaltung der Erfindung ist vorgesehen, dass die wenigstens eine thermoresponsive Verbindung im ersten Temperaturbereich zumindest im Wesentlichen farblos und/oder zumindest überwiegend transparent ist und/oder dass die wenigstens eine thermoresponsive Verbindung im zweiten Temperaturbereich farbig und/oder zumindest überwiegend intransparent ist. Mit anderen Worten ist es vorgesehen, dass die wenigstens eine thermoresponsive Verbindung im ersten Temperaturbereich, der als damit als tolerierbar und risikofrei oder zumindest risikoarm angesehen wird, möglichst keine optische Beeinflussung durch eine Farbigkeit zumindest im für den Menschen sichtbaren Wellenlängenbereich und/oder durch eine verringerte Transmission verursacht. Hierdurch wird ein Chirurg während der Augenoperation nicht beeinträchtigt, solange sich die Temperatur in einem vertretbaren Bereich bewegt bzw. solange die Temperaturschwelle zwischen erstem und zweitem Temperaturbereich nicht überschritten wird. Sobald diese Temperaturschwelle aber überschritten wird, erfolgt sofort eine optische Warnung, indem die wenigstens eine thermoresponsive Verbindung eine Farbe annimmt bzw. ihre Farbe ändert und/oder zumindest überwiegend intransparent wird und damit ein eindeutiges Warnsignal an den behandelnden Arzt sendet, dass temperatursenkende Maßnahmen erforderlich sind.

In einer weiteren vorteilhaften Ausgestaltung der Erfindung ist vorgesehen, dass der vorbestimmte Wellenlängenbereich zwischen 50 µm und 200 nm liegt, insbesondere zwischen 780 nm und 315 nm. Mit anderen Worten ist es erfindungsgemäß vorgesehen, dass die wenigstens eine thermoresponsive Verbindung ihre Farbe und/oder ihren Transmissionsgrad temperaturabhängig im Bereich UV-B, UV-A, für den Menschen sichtbares Licht, nahes Infrarot (IR A, IR B) und mittleres Infrarot (IR C) diskontinuierlich ändert. Eine Änderung im sichtbaren Bereich (ca. 380 nm - 780 nm) hat den Vorteil, dass sie unmittelbar und ohne zusätzliche Hilfsmittel durch einen behandelnden Arzt erkennbar ist. Eine Änderung im IR- oder UV-Bereich kann demgegenüber mithilfe einer geeigneten Detektionsvorrichtung erkannt werden und besitzt den Vorteil, dass die Änderung der optischen Eigenschaft keine Auswirkung im sichtbaren Bereich besitzt und ein Chirurg die Operation ungestört fortsetzen kann.

Besonders bevorzugt beträgt der vorbestimmte Wellenlängenbereich 780 nm, 775 nm, 770 nm, 765 nm, 760 nm, 755 nm, 750 nm, 745 nm, 740 nm, 735 nm, 730 nm, 725 nm, 720 nm, 715 nm, 710 nm, 705 nm, 700 nm, 695 nm, 690 nm, 685 nm, 680 nm, 675 nm, 670 nm, 665 nm, 660 nm, 655 nm, 650 nm, 645 nm, 640 nm, 635 nm, 630 nm, 625 nm, 620 nm, 615 nm, 610 nm, 605 nm, 600 nm, 595 nm, 590 nm, 585 nm, 580 nm, 575 nm, 570 nm, 565 nm, 560 nm, 555 nm, 550 nm, 545 nm, 540 nm, 535 nm, 530 nm, 525 nm, 520 nm, 515 nm, 510 nm, 505 nm, 500 nm, 495 nm, 490 nm, 485 nm, 480 nm, 475 nm, 470 nm, 465 nm, 460 nm, 455 nm, 450 nm, 445 nm, 440 nm, 435 nm, 430 nm, 425 nm, 420 nm, 415 nm, 410 nm, 405 nm, 400 nm, 395 nm, 390 nm, 385 nm, 380 nm, 375 nm, 370 nm, 365 nm, 360 nm, 355 nm, 350 nm, 345 nm, 340 nm, 335 nm, 330 nm, 325 nm, 320 nm oder 315 nm, wobei sämtliche Zwischenwerte und -bereiche als mitoffenbart anzusehen sind.

Ebenfalls offenbart ist eine Detektionsvorrichtung für die Kataraktchirurgie. Die nichterfindungsgemäße Detektionsvorrichtung umfasst wenigstens ein optisches Sensorsystem, mittels welchem eine diskontinuierliche Änderung einer physikalischen Eigenschaft aus der Gruppe Farbe und Transmissionsgrad einer ophthalmologischen Zusammensetzung (OVD) gemäß dem ersten Erfindungsaspekt detektierbar ist, und wenigstens eine zum Datenaustausch mit dem Sensorsystem gekoppelte Mensch-Maschinen-Schnittstelle, mittels welcher im Fall einer Detektion einer diskontinuierlichen Änderung der Farbe und/oder Transparenz der OVD ein optischer und/oder akustischer und/oder haptischer Hinweis an einen Benutzer erzeugbar ist. Hierdurch kann das Risiko einer kornealen Wundverbrennung während einer Augenoperation erheblich verringert werden, da dem Chirurg eine mittels der Detektionsvorrichtung detektierte Farb- und/oder Transparenzänderung mit Hilfe der Mensch-Maschinen-Schnittstelle unmittelbar signalisiert werden kann. Der Chirurg kann dann sofortige Gegenmaßnahmen einleiten, um die Temperatur auf ein akzeptables Niveau zu senken. In Abhängigkeit der Ausgestaltung der OVD kann das Sensorsystem auf unterschiedliche Wellenlängen bzw. Wellenlängenbereiche angepasst sein, um das thermochrome bzw. thermotrope Verhalten der thermoresponsiven Verbindung zu überwachen. Beispielsweise kann das Sensorsystem zur Überwachung im Bereich UV-B und/oder UV-A, im Bereich des für den Menschen sichtbaren Lichts, im nahen Infrarot (IR A, IR B) und/oder im mittleren Infrarot (IR C) ausgebildet sein. Auch beliebige Kombinationen hieraus sind denkbar. Die Detektionsvorrichtung kann grundsätzlich als autarkes, eigenständiges Gerät ausgebildet sein. Vorzugsweise sind alle Elemente der Detektionsvorrichtung dabei in einem gemeinsamen Gehäuse angeordnet. Alternativ können bestimmte Elemente der Detektionsvorrichtung voneinander beabstandet sein. Beispielsweise kann ein Datenaustausch zwischen dem Sensorsystem und Mensch-Maschinen-Schnittstelle kabelgebunden und/oder drahtlos erfolgen. Alternativ kann die Detektionsvorrichtung teilweise oder vollständig in ein anderes Gerät integriert sein bzw. bereits vorhandene Vorrichtungen mitverwenden. Beispielsweise kann als Sensorsystem eine in einem OP-Mikroskop bereits vorhandene Kamera vorteilhaft zur Akquise von Bilddaten verwendet werden. Ebenso kann eine bereits vorhandene Recheneinrichtung vorteilhaft zur Auswertung und Prüfung der Bilddaten des Sensorsystems und/oder zur Steuerung oder Regelung der Mensch-Maschinen-Schnittstelle in Abhängigkeit des Prüfergebnisses der Bilddaten verwendet werden.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind. Es sind auch Ausführungen und Merkmalskombinationen als offenbart anzusehen, die somit nicht alle Merkmale eines ursprünglich formulierten unabhängigen Anspruchs aufweisen. Es sind darüber hinaus Ausführungen und Merkmalskombinationen, insbesondere durch die oben dargelegten Ausführungen, als offenbart anzusehen, die über die in den Rückbezügen der Ansprüche dargelegten Merkmalskombinationen hinausgehen oder von diesen abweichen. Dabei zeigt:
- Fig. 1: schematische Ansichten einer erfindungsgemäßen ophthalmologischen Zusammensetzung (OVD) bei unterschiedlichen Temperaturen, wobei die OVD ein viskoelastisches Polymer und eine thermoresponsive Verbindung umfasst, die ihre Farbe im sichtbaren Wellenlängenbereich temperaturabhängig diskontinuierlich ändert;
- Fig. 2: schematische Ansichten der erfindungsgemäßen OVD bei unterschiedlichen Temperaturen, wobei die OVD ein viskoelastisches Polymer und eine thermoresponsive Verbindung umfasst, welche ihre Farbe im infraroten Wellenlängenbereich temperaturabhängig diskontinuierlich ändert;
- Fig. 3: schematische Ansichten der erfindungsgemäßen OVD bei unterschiedlichen lokalen Temperaturen, wobei die OVD ein viskoelastisches Polymer und eine thermoresponsive Verbindung umfasst, die in Form von Mikrokügelchen im viskoelastischen Polymer vorliegen;
- Fig. 4: ein erstes Konzept zur Herstellung einer thermoresponsiven OVD durch Vernetzung einer thermoresponsiven Verbindung mit einem viskoelastischen Polymer;
- Fig. 5: ein zweites Konzept zur Herstellung einer thermoresponsiven OVD durch Vernetzung von Mikrokügelchen einer thermoresponsiven Verbindung mit einem viskoelastischen Polymer;
- Fig. 6: ein Mechanismus einer direkten Bindung einer thermoresponsiven Verbindung an Hyaluronsäure mittels EDC/NHS-vermittelter Peptidkopplung;
- Fig. 7: ein Mechanismus einer direkten Bindung einer thermoresponsiven Verbindung an Hyaluronsäure durch nukleophile Substitution;
- Fig. 8: ein Mechanismus zur Implementierung von Spacern zwischen der thermoresponsiven Verbindung und Hyaluronsäure; und
- Fig. 9: eine Prinzipdarstellung einer Detektionsvorrichtung für eine Augenoperation.

### Bevorzugte Ausführung der Erfindung

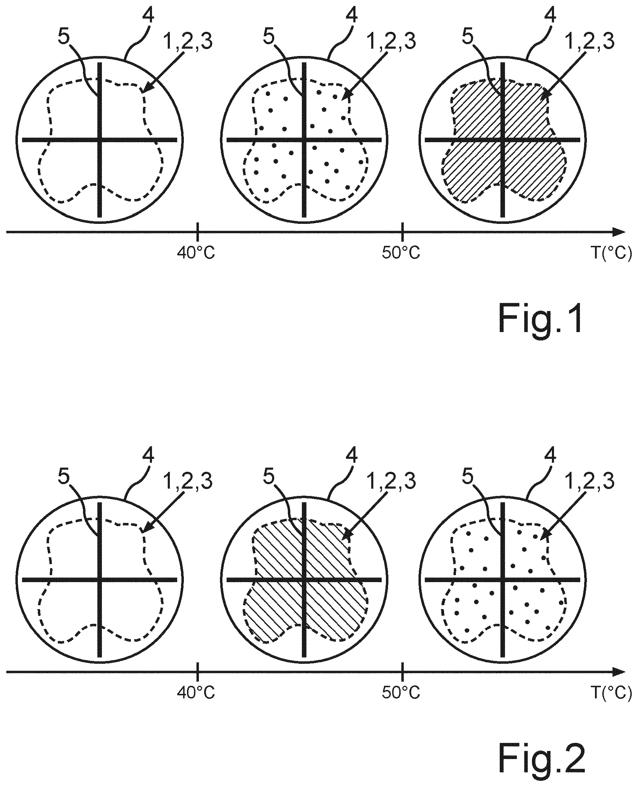

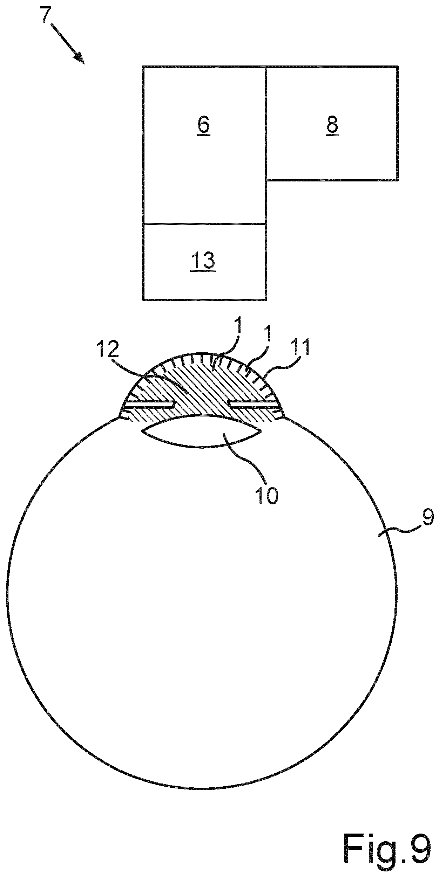

Fig. 1 zeigt drei schematische Ansichten einer erfindungsgemäßen ophthalmologischen Zusammensetzung (OVD) 1 bei etwa 35 °C, etwa 45 °C und etwa 55 °C im Temperaturbereich zwischen etwa 30 °C und 60 °C. Die OVD 1 umfasst ein viskoelastisches Polymer 2, in dem eine thermoresponsive Verbindung 3 gleichmäßig verteilt vorliegt. Die thermoresponsive Verbindung 3 besitzt im vorliegenden Ausführungsbeispiel thermochrome Eigenschaften und ändert ihre Farbe im für den Menschen sichtbaren Wellenlängenbereich temperaturabhängig diskontinuierlich. Zur Verdeutlichung ist die OVD 1 in einem ein Operationsfeld symbolisierenden Probengefäß 4, das ein kartesisches Koordinatensystem 5 enthält, dargestellt. Man erkennt, dass die OVD 1 unterhalb von 40°C, beispielsweise bei 35 °C, farblos und hochtransparent ist, sich bei etwa 45 °C geringfügig färbt und bei etwa 55 °C eine diskontinuierlichen Farbänderung durchlaufen hat, die mit dem bloßen Auge oder mit einem entsprechenden Sensorsystem 6 (s. Fig. 9) detektierbar ist.

Fig. 2 zeigt schematische Ansichten der erfindungsgemäßen OVD 1 bei etwa 35 °C, etwa 45 °C und etwa 55 °C im Temperaturbereich zwischen etwa 30 °C und 60 °C. Im Unterschied zum vorhergehenden Ausführungsbeispiel ändert die thermoresponsive Verbindung 3 ihre Farbe im nah-infraroten Wellenlängenbereich temperaturabhängig diskontinuierlich. Unter 40 °C ist die OVD 1 erneut farblos und hochtransparent und färbt sich diskontinuierlich oberhalb von etwa 40 °C ein, wobei die OVD 1 mit zunehmender Temperatur längere Wellenlängen als das sichtbare Licht emittiert. Diese Farbänderung ist daher in der Regel nicht mit dem Auge, aber mit einem entsprechenden Sensorsystem 6 detektierbar.

Die eine oder mehreren thermoresponsiven Verbindungen 3 innerhalb der OVD 1 sind vorzugsweise so konzipiert, dass sie beim Überschreiten einer definierten Temperaturschwelle ihr Aussehen entweder von farblos zu farbig (Thermochromie) und/oder von transparent zu intransparent (Thermotropie) verändern. Zusätzlich zu einem Wechsel von farblos zu farbig bzw. von transparent zu intransparent können auch temperaturabhängig mehrfache Farb- und/oder Transparenzveränderungen vorgesehen werden. Der Grad der Färbung/Transparenz der thermoresponsiven OVD 1 bietet dem Chirurgen die Möglichkeit, die intraokulare Temperatur anzuzeigen bzw. beim Überschreiten einer Temperaturschwelle durch eine Erhöhung der intraokularen Temperatur einen schnellen Umschwung zwischen einer geringer und einer starken Färbung und/oder einer hohen und einer geringen Transparenz zu zeigen. Die für die Veränderung des Aussehens bestimmte Temperatur wird auf einen bestimmten Schwellenwert, z. B. 40 °C, optimiert. Für die menschliche Hornhaut unbedenkliche Temperaturwerte liegen generell im Bereich von etwa 32 °C bis etwa 38 °C. Als kritische Temperatur für eine Hornhautverbrennung wird generell 60 °C angesehen.

Eine Möglichkeit, eine thermoresponsive OVD 1 herzustellen, ist die Modifikation eines viskoelastischen Polymers mit thermoresponsiven Partikeln. Dies kann beispielsweise durch die Einbindung von Farbstoffen als thermochrome Verbindung 3 in ein Polymernetzwerk erreicht werden. Diese Farbstoffe 3 können reversibel stabile delokalisierte Elektronensysteme bilden, die in der Lage sind, Licht im visuellen Spektrum zu absorbieren wenn die Temperatur steigt, beispielsweise durch reversible Ringschlussreaktionen. Vorzugsweise wird hierbei ein Farbstoff 3 verwendet, der im fernen oder nahen Infrarot-Spektrum emittiert, solange die Temperatur unter einer kritischen Temperaturschwelle liegt, und der im für den Menschen sichtbaren Spektrum (oder im nahen Infrarot-Spektrum) emittiert, wenn die Temperatur über der vorbestimmten Temperaturschwelle liegt (vgl. Fig. 1, Fig. 2). Vorzugsweise besitzt der Farbstoff 3 nicht in beiden Zuständen eine sichtbare Lichtemission, da dies eine dauerhaft gefärbte OVD 1 erzeugen würde, die die Sicht eines Chirurgen während einer Kataraktoperation beeinträchtigen könnte.

Alternativ oder zusätzlich zu einer thermochromen Verbindung 3 können auch thermotrope Verbindungen 3 verwendet werden, die sich beim Überschreiten einer vorbestimmten Temperaturschwelle eintrüben. Experimentell kann dies beispielsweise durch Turbidimitrie untersucht werden, wobei im Rahmen der vorliegenden Offenbarung die Temperatur bei einer definierten Transmission von 50 % als Temperaturschwelle anzusehen ist.

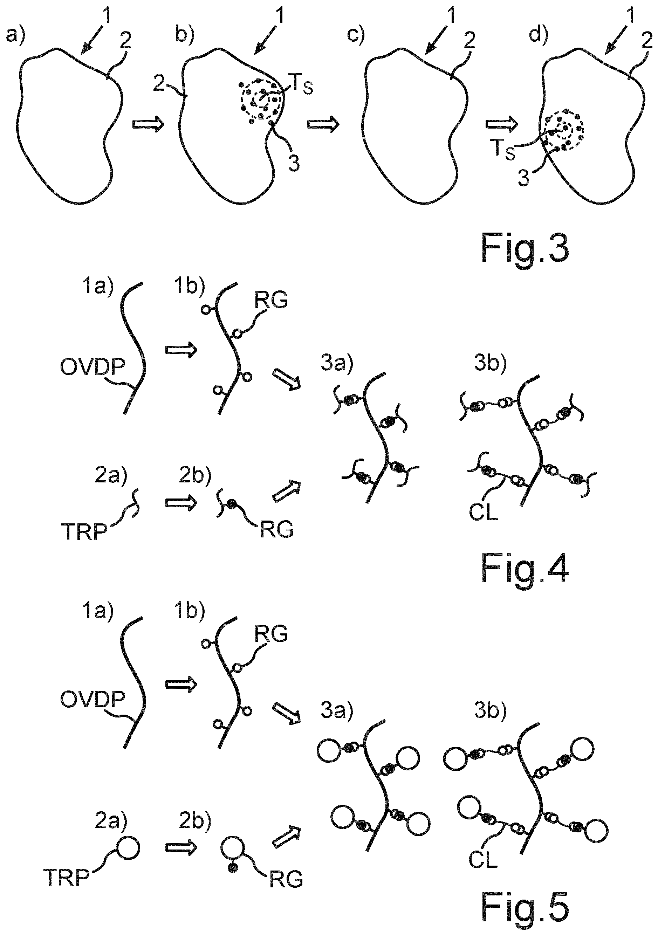

Fig. 3 zeigt vier schematische Ansichten der erfindungsgemäßen OVD 1 bei unterschiedlichen lokalen Temperaturen. Dabei liegt die thermoresponsive Verbindung 3 in Form von Mikrokügelchen (microbeads) im viskoelastischen Polymer 2 verteilt vor. Die Mikrokügelchen 3 können kovalent an das Polymer 2 angebunden sein. Unterhalb ihrer Phasenübergangstemperatur sind die Mikrokügelchen 3 unsichtbar (a). Wenn eine Wärmequelle die Temperatur lokal über die Phasenübergangs- oder Schwellentemperatur Ts erwärmt, führen diese Mikrokügelchen 3 einen Phasenübergang durch und werden sichtbar (b). Nachdem die Temperatur wieder unter die Phasenübergangstemperatur Ts gesunken ist, werden die Mikrokügelchen 3 wieder unsichtbar (c). Wenn die Temperatur lokal an einem anderen Ort über die Phasenübergangstemperatur Ts steigt, werden die Mikroperlen 3 am betreffenden Ort wieder sichtbar (d).

Zusätzlich zu oder anstelle von kurzkettigen oder in Form von Mikroperlen vorliegenden Verbindungen 3 können auch thermoresponsive Polymere vorgesehen sein, die ihre optischen Eigenschaften bei Erreichen einer bestimmten Temperaturschwelle reversibel umschalten. Geeignete Polymere umfassen beispielsweise Wiederholeinheiten aus Poly(N-Isopropylacrylamid) (PNIPAM) oder einer Vielzahl ähnlicher Copolymere wie beispielsweise Poly(ethylenglycol) (PEG) und/oder Poly(ε-Caprolacton) (PCL). Während ihres Phasenübergangs durchlaufen solche Polymere einen Wechsel zwischen vollständig transparent und überwiegend oder vollständig undurchsichtig. Das bedeutet, dass sie unterhalb ihrer Phasenübergangstemperatur Ts für das Auge unsichtbar sind und oberhalb der Phasenübergangstemperatur Ts sichtbar werden. Darüber hinaus ist es auch möglich, mit diesen Polymeren Mikropartikel oder andere kleinskalige Strukturen zu erzeugen, die möglichst schnelle Phasenübergänge durchlaufen können. Weiterhin ist es möglich, reaktive Gruppen in diese Polymere einzubauen, die zur chemischen Verbindung mit viskoelastischen Polymerketten innerhalb der OVD 1 verwendet werden könnten. Als Ergebnis besitzt die resultierende OVD 1 sozusagen lokalisierte thermoresponsive Sensoren, die in der Lage sind, einen lokalen Temperaturanstieg über einen bestimmten Schwellenwert hinaus zu erkennen.

Dies bietet den Vorteil, dass diese Mikrokugeln mit dem bloßen Auge völlig unsichtbar sind, solange die Temperatur unterhalb der Phasenübergangstemperatur für diese Polymere liegt. Das bedeutet, dass der Chirurg die Operation ohne Sehbehinderungen durch die "Sensoren" 3 durchführen kann, solange die intraokulare Temperatur unter der kritischen Schwelle liegt. Sobald die Temperatur ansteigt, würde der Phasenübergang der thermoresponsiven Partikel 3 mit dem menschlichen Auge im erwärmten Bereich sichtbar werden und den Arzt vor einem Temperaturanstieg warnen. Alternativ kann der Phasenübergang der thermoresponsiven Partikel 3 über einen in das Operationsmikroskop integrierten bildgebenden Sensor erfasst werden. Eine hochentwickelte bildgebende Kamera hat den Vorteil einer höheren Empfindlichkeit im Vergleich zum Auge des Chirurgen. Durch das Okular eines Operationsmikroskops und/oder auf einem digitalen Bildschirm als Mensch-Maschine-Interface kann eine Echtzeit-Überlagerung des Operationsbildes und des gegebenenfalls verstärkten detektierten Farbsignals aus der thermoresponsiven OVD 1 Informationen über die Temperaturverteilung innerhalb des Operationsfeldes liefern. Diese Option ermöglicht auch die Erkennung eines schwachen OVD-Farb-/Transparenzsignals und kann die Reaktionszeit des Chirurgen verkürzen. Grundsätzlich ist es möglich, eine Schnittstelle zwischen dem Operationsmikroskop und der Phakomaschine vorzusehen, so dass gegebenenfalls eine Steuerung und/oder Regelung der Parameter der Phakomaschine zur automatischen Anpassung der aktuell zugeführten Phakoenergie und/oder der Irrigations- und/oder Aspirationsrate erfolgen kann.

Im Folgenden werden verschiedene Herstellungsmöglichkeiten für thermoresponsive OVDs 1 auf der Basis von thermoresponsiven Polymeren 2 dargestellt. Die verwendeten thermoresponsiven Verbindungen 3 sollten vorzugsweise ungiftig bzw. toxikologisch tolerierbar sein, einen Mechanismus zur Bindung an viskoelastische Polymere 2 bieten und einen diskontinuierlichen Farb- und/oder Transparenzübergang bei einer definierten Temperaturschwelle bzw. innerhalb eines möglichst engen Temperaturbereichs (z. B. innerhalb von 5-10 K) besitzen.

Verschiedene thermoresponsive Polymere 2 sind an sich bekannt und wurden bereits in unterschiedlichen Varianten hergestellt, zum Beispiel Poly(N-isopropylacrylamid) (PNIPAM), Poly[2-(dimethylamino)ethylmethacrylat], Hydroxypropylcellulose oder Polyvinylmethylether. Die populärste Gruppe dieser Polymere basiert auf PNIPAM mit der allgemeinen Formel I:

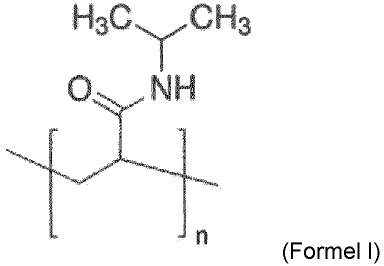

Da die Herstellung von PNIPAM auf der Acrylatpolymerisation basiert, existieren viele Möglichkeiten, Copolymere auf der Basis dieses Acrylamids mit einer Vielzahl unterschiedlicher Phasenübergangstemperaturen herzustellen. Der grundlegende Mechanismus für den Phasenübergang in thermoresponsiven Polymeren ist in allen Fällen der gleiche, nämlich eine drastische Verschiebung der thermodynamischen Wechselwirkungen zwischen Polymer und Wasser in Abhängigkeit von der Temperatur. Unterhalb der Phasenübergangstemperatur ist es für die Polymerkette thermodynamisch günstiger, mit den umgebenden Wassermolekülen zu interagieren. In diesem Zustand liegen die Polymerketten als langgestreckte Kette vor und sind für das Auge unsichtbar. Oberhalb der Phasenübergangstemperatur wird es für die Polymerketten günstiger, mit sich selbst zu interagieren und Wasser aus ihrem Netzwerk auszuschließen. In diesem Zustand liegen sie als kollabierte Polymerketten vor und sind in der Lage, Licht zu streuen. Aufgrund dieser Wechselwirkung ist eine wässrige Lösung von PNIPAM oder ähnlichen Polymeren unterhalb der Phasenübergangstemperatur transparent und wird oberhalb dieser Temperatur undurchsichtig.

Die Phasenübergangstemperatur kann durch die Einführung anderer Monomertypen, beispielsweise von Acrylaten und/oder Acrylamiden moduliert werden, um maßgeschneiderte Copolymere zu erzeugen. Dadurch können thermoresponsive Verbindungen 3 erzeugt werden, die in der Lage sind, verschiedene Temperaturschwellenwerte "zu erkennen". Darüber hinaus können diese Verbindungen 3 in verschiedene Strukturen wie makroskopische Gele oder Mikropartikel eingebracht werden. Ebenso kann eine Einkapselung vorgesehen sein. Darüber hinaus erlaubt die Copolymerisation die Einführung anderer reaktiver Gruppen, die zur kovalenten Bindung solcher thermoresponsiven Verbindungen 3 an andere Trägermoleküle, wie z. B. Hyaluronsäure oder andere viskoelastischen Polymere, genutzt werden können.

Im Folgenden werden exemplarisch zwei Möglichkeiten vorgestellt, wie thermoresponsive OVDs 1 realisiert werden können. Eine erste, in Fig. 4 gezeigte Möglichkeit besteht im kovalenten Binden thermoresponsiver Polymerstränge TRP an viskoelastische Polymere OVDP. Eine zweite, in Fig. 5 gezeigte Möglichkeit besteht in der kovalenten Verbindung thermoresponsiver Mikrokugeln TRP mit viskoelastischen Polymeren OVDP. Es ist an dieser Stelle zu betonen, dass die Erfindung nicht auf diese Ausführungsformen beschränkt ist, da es eine große Vielfalt möglicher Polymere bzw. Copolymere und Herstellungsmöglichkeiten gibt, um eine erfindungsgemäße OVD 1 zu realisieren.

Wie zuvor beschrieben, besteht eine mögliche Strategie zur Herstellung einer thermoresponsiven OVD 1 darin, die viskoelastischen Polymere 2 mit thermoresponsiven Verbindungen 3 zu modifizieren. Dies kann mit dem folgenden allgemeinen Ansatz realisiert werden:
1) Modifizierung der viskoelastischen Polymere (OVDP) 2 der OVD 1 mit reaktiven Gruppen (Fig. 4, Fig. 5: 1b).
2) Modifizierung der "thermoresponsiven Polymere" (TRP) 3 mit reaktiven Gruppen RG (Fig. 4, Fig. 5: 2b), die im Fall komplementärer RGs eine direkte Verknüpfung von TRP 3 mit den OVDP 2 erlauben (Fig. 4, Fig. 5: 3a).
3) Kovalentes Verbinden der reaktiven Gruppen RG der OVDPs 2 mit reaktiven Gruppen RG der TRPs 3 über einen entsprechenden Quervernetzer CL (Fig. 4, Fig. 5: 3b). Die reaktiven Gruppen RG können generell unabhängig voneinander ausgewählt werden und können gleich oder unterschiedlich sein. Die TRPs 3 können je nach späterem Einsatzzweck der OVD 1 als einzelne Polymerketten (Fig. 4) oder in Form von Polymermikropartikeln (Fig. 5) gebunden werden.

Im ersten Ansatz werden einzelne Stränge der TRPs 3 kovalent mit den OVDPs 2 verknüpft. Dies kann durch die Modifizierung beider Edukte mit chemisch reaktiven Gruppen RG erreicht werden. Wenn diese Gruppen RG komplementär zueinander sind, können sie direkt miteinander reagieren und eine direkte Bindung der TRPs 3 an die OVDPs 2 ermöglichen (Fig. 4, Fig. 5: 3a). Die TRPs 3 können dann bei Erreichen ihrer Schwellentemperatur einen Phasenübergang durchlaufen und eine lokale Trübung und/oder Färbung erzeugen. Dies ist der einfachste Weg, um eine thermoresponsive OVD 1 herzustellen. Der kurze Abstand zwischen den beiden Edukten kann jedoch sterische bzw. physikalische Wechselwirkungen erzeugen, die die Fähigkeit der TRPs 3 zum temperaturabhängigen Phasenübergang beeinträchtigen. Daher können in einer alternativen Ausführung Quervernetzer CL eingesetzt werden, um die TRPs 3 in einem größeren Abstand zu den OVDPs 2 zu verankern. Abhängig von der gewählten Länge der Quervernetzer CL können sich die TRP-Ketten 3 dann unabhängiger von den OVDPs 2 verhalten.

Eine Alternative ist, wie bereits erwähnt, die Verwendung von TRP-Mikropartikeln anstelle von einzelnen TRP-Strängen. Diese Entitäten können sehr schnelle Phasenübergänge durchlaufen, was vorteilhaft ist, um eine schnelle Erkennung zu ermöglichen, ob im Rahmen einer Augenoperation eine vorbestimmte Temperaturschwelle überschritten wurde. Je nach Größe und Verteilung dieser Partikel besteht die visuelle Reaktion beim Erreichen einer Temperaturschwelle nicht in einer allgemeinen Eintrübung bzw. Färbung der erhitzten Stelle, sondern im Auftreten kleiner "Punkte", wie dies in Fig. 3 gezeigt ist. Dies stellt in manchen Fällen ein wünschenswerteres visuelles Feedback für Ärzte dar, das sich weniger störend auf die Operation auswirkt. Allerdings wird der Herstellungsprozess dadurch etwa komplexer, da zunächst die Mikropartikel aus einzelnen Stränge von TRP synthetisiert werden müssen.

Häufig werden als viskoelastischen Polymere 2 für OVDs 1 Verbindungen aus der Gruppe der Polysaccharide verwendet. Als besonders geeignet hat sich dabei Hyaluronsäure (HA, Formel II a)) gezeigt, bei der es sich um ein sehr häufig verwendetes Biopolymer für OVDs 1 handelt. Ein großer Vorteil dieser Gruppe von Makromolekülen ist ihre Fähigkeit, aufgrund der Existenz von Carboxylfunktionalitäten mit Hilfe gut erforschter und etablierter EDC (1-Ethyl-3-[3-dimethylaminopropyl]carbodiimid)/NHS (N-Hydroxysuccinimid)-Peptidkopplungschemie leicht mit weiteren chemischen Gruppen modifiziert zu werden. Dies ist schematisch in Formel II b) gezeigt:

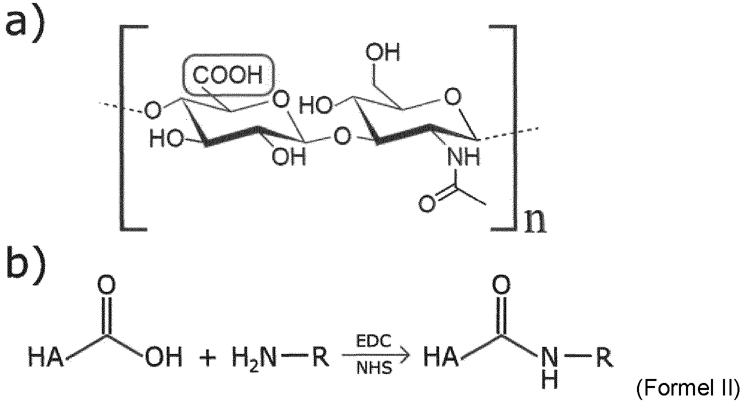

Die reaktive Carboxylgruppe der HA für weitere chemische Modifikationen ist in Formel II a) umrahmt. In Formel II b) ist das Reaktionsschema der EDC/NHS-vermittelten Peptidkopplungsreaktion angegeben. "HA" steht dabei für das Hyaluronsäure-Grundgerüst. "R" ist entweder die reaktive Gruppe, die mit HA verbunden wird, ein Spacer, der direkt zum TRP führt, oder ein Quervernetzer, der anschließend mit dem gegebenenfalls korrespondierend modifizierten TRP verbunden wird.

Dieser Reaktionstyp kann dazu verwendet werden, eine reaktive Gruppe einzuführen, an die ein amidmodifiziertes TRP binden kann. Ebenso kann dieser Reaktionstyp dazu verwendet werden, eine andere reaktive Gruppe an HA zu binden, um anschließend mit dem TRP zu binden oder mit einer dritten Gruppe zu vernetzen.

Daher sind Polysaccharide und insbesondere HA vorteilhafte Kandidaten für diese Ausführungsform. Andere viskoelastische Polymere können jedoch auch auf andere Weise modifiziert, chemisch mit thermoresponsiven Verbindungen 2 gekoppelt und zur Herstellung einer thermoresponsiven OVD 1 verwendet werden.

Wie bereits erwähnt, basiert eine große Gruppe von thermoresponsiven Verbindungen und Polymeren auf PNIPAM. Dementsprechend existiert auch eine große Vielfalt von PNIPAM-Derivaten, Co-Polymeren (einfache Co-Polymere, Triblock-Copolymere etc.), um TRPs mit einer großen Auswahl an Phasenübergangstemperaturen, rheologischen Eigenschaften und sogar einer Anfälligkeit für andere sich ändernde Parameter wie beispielsweise pH-Wert und/oder Salzkonzentration herzustellen.

In einer ersten Ausführungsform wird ein PNIPAM-Copolymer gewählt, um einen möglichen Weg zur Herstellung einer wärmeempfindlichen OVD 1 zu beschreiben. Das Copolymer besteht überwiegend aus N-Isopropylsäure und einem kleinen Anteil (< 1%) einer Acrylsäure, die die notwendige reaktive Gruppe RG zur Bindung an Hyaluronsäure enthält. Der Anteil der Acrylsäure sollte in der Regel so klein wie möglich sein, um die thermosensitiven Eigenschaften von PNIPAM nicht negativ zu beeinflussen und eine mögliche Bindung an mehrere reaktive Gruppen zu vermeiden.

In einer weiteren Ausführungsform können weitere Modifikationen zur Anpassung der Phasenübergangstemperatur vorgenommen werden, da reines PNIPAM eine Phasenübergangstemperatur von etwa 32 °C hat. Dies kann beispielsweise durch Copolymerisation mit anderen Acrylaten und/oder einer Einstellung der Polymerkettenlänge erreicht werden. Zu diesem Zweck ist es vorteilhaft, dass viele unterschiedliche Acrylsäurederivate verfügbar sind, so dass die Acryl-Kettenreaktionspolymerisation die Kombination einer Vielzahl verschiedener Acrylsäurederivate zu Polyacrylaten mit unterschiedlichen Eigenschaften erlaubt.

Es muss jedoch darauf hingewiesen werden, dass diese Erfindung nicht auf PNIPAM und seine Derivate beschränkt ist. Im Wesentlichen kann jedes geeignete Polymer, das bei einer gewünschten Schwellentemperatur einen entsprechenden Phasenübergang zeigt, für diese Erfindung verwendet werden. Vorzugsweise werden thermoresponsive Verbindungen 2 verwendet, die die folgenden Voraussetzungen erfüllen:
- Sie besitzen eine reaktive Gruppe zur Bindung an das OVDP oder können (z. B. durch Kreuzpolymerisation) mit einer solchen reaktiven Gruppe modifiziert werden.
- Sie sind in einer Weise biokompatibel, die das intraokulare Umfeld des Patienten während oder nach der Kataraktoperation nicht schädigt.
- Wenn Mikropartikel verwendet werden sollen, muss die thermoresponsive Verbindung zur Herstellung solcher Partikel geeignet sein.

### Auswahl des Bindungsmechanismus

Wie bei der Auswahl von TRPs existieren zahlreiche chemische Reaktionstypen, die zur Verknüpfung von TRPs mit OVDPs verwendet werden können. Im Folgenden werden einige Reaktionstypen aufgezählt, wobei diese Aufzählung nicht abschließend ist.

Eine besonders vorteilhafte Möglichkeit besteht darin, ein PNIPAM-Copolymer mit einer primären Aminfunktion zu modifizieren und es mit Hilfe einer EDC/NHS-Kopplung direkt an die Carboxylfunktion der HA zu binden.

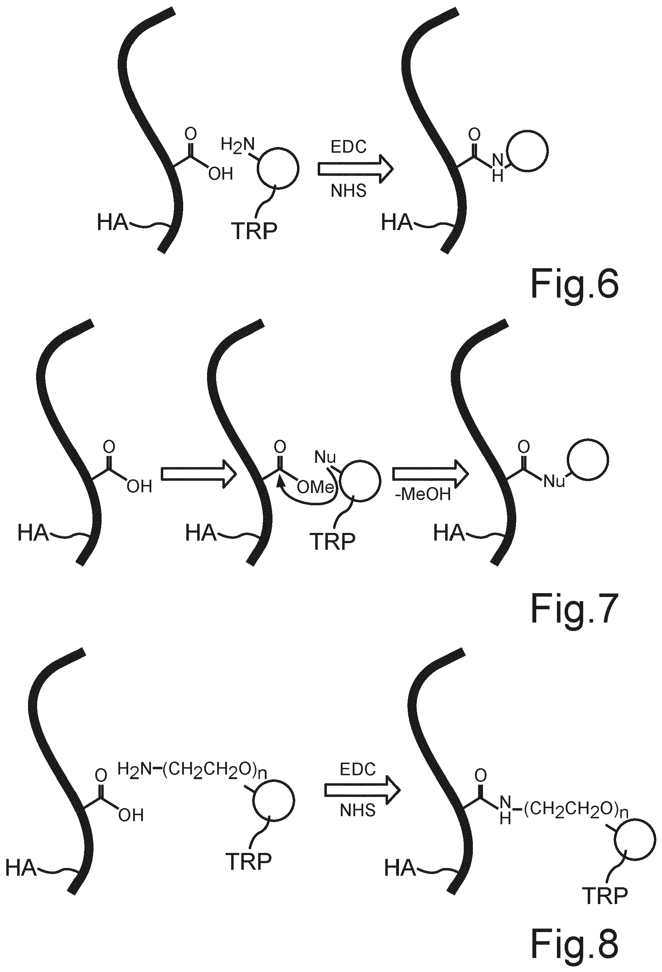

Dieser Mechanismus der direkten Bindung eines TRP an Hyaluronsäure (oder einem anderen geeigneten Polymer) mittels EDC/NHS-vermittelter Peptidkopplung ist in Fig. 6 dargestellt. Die TRP muss gegebenenfalls zunächst mit einem primären Amin modifiziert werden, entweder durch Copolymerisation einer entsprechend modifizierten Acrylsäure, die ein solches primäres Amin trägt, oder durch Funktionalisierung des Polymers mit einer anderen Reaktion. In diesem Zusammenhang wird darauf hingewiesen, dass der Begriff "Acrylsäure" bzw. "Acrylat" im Rahmen der vorliegenden Offenbarung auch Alkylacrylsäuren bzw. Alkylacrylate umfasst, beispielsweise Methacrylsäure bzw. Methacrylat, Ethylacrylsäure, Ethylacrylat etc.

Alternativ kann die Carboxylgruppen von HA (oder einem anderen geeigneten Polymer) in Estergruppen umgewandelt werden, die anschließend über eine nukleophile Substitution mit einer nukleophilen Gruppe des PNIPAM-Copolymers (oder eines anderen TRPs) reagieren. Exemplarisch ist in Fig. 7 ein solcher Mechanismus der direkten Bindung einer TRP an Hyaluronsäure durch nukleophile Substitution gezeigt. Zu diesem Zweck wird zunächst die Carboxylgruppe der HA methoxyliert, was einen späteren Angriff durch eine nukleophile Gruppe des TRP ermöglicht. Durch diesen zusätzlichen Reaktionsschritt kann das TRP flexibler ausgewählt werden, da im Vergleich zu Fig. 6 eine größere Auswahl reaktiver Gruppen als Reaktionspartner in Frage kommt.

Als weitere Alternative kann auch die sogenannte "Click-Chemistry" verwendet werden, um die beiden Edukte miteinander zu verbinden, wie z. B. Michael Addition oder Diels-Alder-Addition. Da sowohl die Hyaluronsäure (oder andere viskoelastische Polymere) als auch die PNIPAM-Copolymere (oder andere thermoresponsive Verbindungen) leicht modifizierbar sind, ist es möglich, eine Vielzahl reaktiver Gruppen einzuführen und auf eine große Anzahl möglicher Verknüpfungsreaktionen zuzugreifen.

Um den Abstand zwischen dem viskoelastischen Polymer und der thermoresponsiven Verbindung zu vergrößern, können die gleichen chemischen Reaktionstypen für die Kopplung von Spacern verwendet werden. Dies kann entweder durch Verwendung als Quervernetzer oder durch Bindung an die HA oder TRP vor der Reaktion erfolgen. Ein Beispiel für Spacer wäre funktionalisiertes Polyethylenglykol (PEG), das mit einer Vielzahl möglicher funktioneller Gruppen gut erforscht ist. Darüber hinaus ist allgemein bekannt, dass PEG ein sehr günstiges Toxizitätsprofil aufweist.

In Fig. 8 ist eine Methode zur Implementierung von Spacern zwischen dem TRP und der Hyaluronsäure zur Vergrößerung des Abstands dargestellt. Das TRP wird mit einem Spacer modifiziert, der ein endständiges primäres Amin trägt, das dann in der bereits beschriebenen Weise an die Carboxylgruppe der Hyaluronsäure gekoppelt werden kann. Dies kann erreicht werden, indem man ein PEG mit einer Acrylsäurefunktion an einem Ende und einem primären Amin am anderen Ende der Polymerkette erzeugt und dieses anschließend mit dem TRP copolymerisiert.

Auch hier ist festzuhalten, dass die EDC/NHS-vermittelte Peptidkopplung in den Beispielen zur Bindung einer TRP an Hyaluronsäure zwar bevorzug verwendet wird, es sich aber nur um eine von vielen möglichen Verfahren handelt, wie dies erreicht werden kann. Das Gebiet der Chemie verfügt über eine große Anzahl anderer möglicher Mechanismen.

Die zusätzliche Eigenschaft der Thermoresponsivität ist generell für alle OVD-Typen anwendbar. Sie bietet ein hohes Potenzial zur Verringerung des Risikos einer thermischen Verletzung der Augenstrukturen. Besonders große Vorteile ergeben sich für dispersive OVDs, die normalerweise während der Operation zum Schutz des nicht regenerativen Hornhautendothels eingesetzt werden. Ein intraoperatives chirurgisches Trauma kann eine irreversible Endothelbeschädigung verursachen. Da eine Wundheilung oder Regeneration des Hornhautendothels nicht stattfindet, bietet eine dispersive OVD 1 mit thermoresponsiven Eigenschaften sowohl einen Beschichtungsschutz des Hornhautendothels als auch einen Temperatursensor am Hornhautendothel. Damit liefert sie wichtige Informationen über Temperaturänderungen an dieser empfindlichen Zellschicht.

Mit Hilfe der vorgeschlagenen Erfindung kann zudem eine automatische Erkennung der OVD-Färbung/Trübung über einen beispielsweise im Operationsmikroskop integrierten bildgebenden Sensor die Ausgabe eines Warnsignals mittels einer Mensch-Maschine-Schnittstelle 8 (Fig. 9) ermöglichen, wenn die erfasste Temperatur über einer kritischen Grenztemperatur liegt. Darüber hinaus kann eine automatische Anpassung der Parameter einer Phakomaschine vorgesehen sein, wenn eine entsprechende kabelgebundene und/oder drahtlose Schnittstelle zwischen dem optischen Sensorsystem 6 bzw. der Mensch-Maschine-Schnittstelle 8 und der Phakomaschine vorhanden ist.

Fig. 9 zeigt hierzu eine Prinzipdarstellung einer nicht-erfindungsgemäßen Detektionsvorrichtung 7 für eine Operation an einem menschlichen oder tierischen Auge 9, wobei es sich bei der Operation exemplarisch um eine Phakoemulsifikation einer Linse 10 zur Behandlung des grauen Stars (Katarakt) handelt. Die Detektionsvorrichtung 7 umfasst wenigstens ein optisches Sensorsystem 6, mittels welchem eine diskontinuierliche Änderung einer physikalischen Eigenschaft aus der Gruppe Farbe und Transmissionsgrad einer erfindungsgemäßen thermoresponsiven OVD 1 detektierbar ist. Das Sensorsystem 6 ist im gezeigten Ausführungsbeispiel in ein Operationsmikroskop integriert, kann aber grundsätzlich auch als separates System vorliegen oder in einem anderen System integriert sein. Weiterhin umfasst die Detektionsvorrichtung 7 wenigstens eine zum Datenaustausch mit dem Sensorsystem 6 gekoppelte Mensch-Maschinen-Schnittstelle 8, mittels welcher im Fall einer Detektion der diskontinuierlichen Änderung der optischen Eigenschaften der OVD 1 ein optischer und/oder akustischer und/oder haptischer Hinweis an einen Benutzer erzeugbar ist. Gegebenenfalls kann die Detektionsvorrichtung 7 einen grundsätzlich optionalen optischen Filter 13 aufweisen, um die Detektion der OVD 1 zu verbessern.

Man erkennt, dass die erfindungsgemäße thermoresponsive OVD 1 nicht nur die Anwendung an der vorderen Hornhautoberfläche (wenn sie z. B. von außen auf die Hornhaut 11 aufgebracht wird) ermöglicht, sondern auch an den intraokularen Strukturen (wenn sie z. B. in die Vorderkammer 12 eingebracht wird). Die OVD 1 kann in Spritzen geliefert werden, die mit einem Luer-Lock-Sicherheitssystem ausgestattet sind. Aber natürlich sind auch andere Liefer- und Applizierungsformen denkbar.

Die in den Unterlagen angegebenen Parameterwerte zur Definition von Prozess- und Messbedingungen für die Charakterisierung von spezifischen Eigenschaften des Erfindungsgegenstands sind auch im Rahmen von Abweichungen - beispielsweise aufgrund von Messfehlern, Systemfehlern, DIN-Toleranzen und dergleichen - als vom Rahmen der Erfindung mitumfasst anzusehen.

### Bezugszeichenliste

- 1: ophthalmologische Zusammensetzung (OVD)
- 2: viskoelastisches Polymer (OVDP)
- 3: thermoresponsive Verbindung (TRP)
- 4: Probengefäß
- 5: Koordinatensystem
- 6: Sensorsystem
- 7: Detektionsvorrichtung
- 8: Mensch-Maschine-Schnittstelle
- 9: Auge
- 10: Linse
- 11: Hornhaut
- 12: Vorderkammer
- 13: Filter
- HA: Hyaluronsäure
- RG: reaktive Gruppe
- CL: Quervernetzer
- Ts: Temperaturschwelle
- Nu: Nukleophil

## Patentansprüche

1. Ophthalmologische Zusammensetzung (1), umfassend wenigstens ein viskoelastisches Polymer (2),
**dadurch gekennzeichnet, dass**
diese wenigstens eine thermoresponsive Verbindung (3) umfasst, welche temperaturabhängig in einem vorbestimmten Wellenlängenbereich wenigstens eine physikalische Eigenschaft aus der Gruppe Farbe und Transmissionsgrad diskontinuierlich ändert, wobei die wenigstens eine thermoresponsive Verbindung (3) in einem ersten Temperaturbereich unterhalb von 60 °C eine erste Farb- und/oder Transmissionseigenschaft und in einem oberhalb des ersten Temperaturbereichs liegenden zweiten Temperaturbereich eine von der ersten Farb- und/oder Transmissionseigenschaft unterschiedliche zweite Farb- und/oder Transmissionseigenschaft besitzt.

2. Ophthalmologische Zusammensetzung (1) nach Anspruch 1,
**dadurch gekennzeichnet, dass**
eine temperaturabhängige Änderung der wenigstens einen physikalischen Eigenschaft reversibel oder irreversibel ist und/oder dass die temperaturabhängige Änderung der wenigstens einen physikalischen Eigenschaft beim Überschreiten eines vorbestimmten Temperaturgrenzwerts innerhalb von höchstens 10 Sekunden, vorzugsweise innerhalb von höchstens 2 Sekunden, erfolgt.

3. Ophthalmologische Zusammensetzung (1) nach Anspruch 1 oder 2,
**dadurch gekennzeichnet, dass**
die wenigstens eine thermoresponsive Verbindung (3) ausgewählt ist aus einer Gruppe, die Polymere, interpenetrierende Polymernetzwerke, semiinterpenetrierende Polymernetzwerke, Flüssigkristalle, Pigmente, Farbstoffe, Tinten, Mikrokapseln und beliebige Kombinationen hieraus umfasst.

4. Ophthalmologische Zusammensetzung (1) nach Anspruch 3,
**dadurch gekennzeichnet, dass**
die wenigstens eine thermoresponsive Verbindung (3) wenigstens eine konstitutionelle Einheit umfasst, die ausgewählt ist aus einer Gruppe, die Poly(N-Isopropylacrylamid), Poly(N,N-Diethylacrylamid), Poly(methylvinylether), Poly(N-Vinylcaprolactam), ein Blockcopolymer aus Poly(ethylenoxid) und Poly(propylenoxid), ein Poly(pentapeptid) von Elastin, ein interpenetrierendes Netzwerk aus Polyacrylamid und Polyacrylsäure, und/oder Copolymerisate hiervon umfasst.

5. Ophthalmologische Zusammensetzung (1) nach einem der Ansprüche 1 bis 4,
**dadurch gekennzeichnet, dass**
das wenigstens eine viskoelastische Polymer (2) ein Polysaccharid umfasst, das aus einer Gruppe ausgewählt ist, die Glykosaminoglykane, Cellulose, ein Celluloseether mit Methyl- und/oder Ethyl- und/oder Propylgruppen, insbesondere Hydroxypropylmethylcellulose, Hydroxyethylmethylcellulose und/oder Methylcellulose, ein Glycosaminoglykan, insbesondere Hyaluronsäure, Chondroitinsulfat, Dermatansulfat, Heparin, Heparansulfat, Keratansulfat, Alginsäure, Polymannuronsäure, Polyguluronsäure, Polyglucuronsäure, Amylose, Amylopektin, Callose, Chitosan, Polygalactomannan, Dextran, Xanthan und/oder eine beliebige Mischung hieraus, Copolymerisate hiervon und pharmakologisch akzeptable Salze hiervon umfasst.

6. Ophthalmologische Zusammensetzung (1) nach einem der Ansprüche 1 bis 5,
**dadurch gekennzeichnet, dass**
die wenigstens eine thermoresponsive Verbindung (3), insbesondere über einen Spacer, kovalent mit dem wenigstens einen viskoelastischen Polymer (2) verbunden ist und/oder dass die wenigstens eine thermoresponsive Verbindung (3) in Form von Partikeln und/oder Mikrokugeln im wenigstens einen viskoelastischen Polymer (2) verteilt vorliegt und/oder dass das wenigstens eine viskoelastische Polymer (2) und die wenigstens eine thermoresponsive Verbindung (3) ein semiinterpenetrierendes und/oder interpenetrierendes Netzwerk bilden.

7. Ophthalmologische Zusammensetzung (1) nach einem der Ansprüche 1 bis 6,
**dadurch gekennzeichnet, dass**
die wenigstens eine thermoresponsive Verbindung (3) im ersten Temperaturbereich unterhalb von 60 °C, insbesondere unterhalb von 40 °C, und oberhalb von 35 °C die erste Farb- und/oder Transmissionseigenschaft und im oberhalb des ersten Temperaturbereichs liegenden zweiten Temperaturbereich die von der ersten Farb- und/oder Transmissionseigenschaft unterschiedliche zweite Farb- und/oder Transmissionseigenschaft besitzt.

8. Ophthalmologische Zusammensetzung (1) nach Anspruch 7,
**dadurch gekennzeichnet, dass**
die wenigstens eine thermoresponsive Verbindung (3) im ersten Temperaturbereich zumindest im Wesentlichen farblos und/oder zumindest überwiegend transparent ist und/oder dass die wenigstens eine thermoresponsive Verbindung (3) im zweiten Temperaturbereich farbig und/oder zumindest überwiegend intransparent ist.

9. Ophthalmologische Zusammensetzung (1) nach einem der Ansprüche 1 bis 8,
**dadurch gekennzeichnet, dass**
der vorbestimmte Wellenlängenbereich zwischen 50 µm und 200 nm liegt, insbesondere zwischen 780 nm und 315 nm.

## Claims

1. Ophthalmological composition (1) comprising at least one viscoelastic polymer (2),
**characterized in that**
this comprises at least one thermoresponsive compound (3) that in a predefined wavelength range undergoes a temperature-dependent discontinuous change in at least one physical property from the group colour and transmittance, wherein the at least one thermoresponsive compound (3) has, in a first temperature range below 60°C, a first colour and/or transmission property and, in a second temperature range that is above the first temperature range, a second colour and/or transmission property that is different from the first colour and/or transmission property.

2. Ophthalmological composition (1) according to Claim 1,
**characterized in that**
a temperature-dependent change in the at least one physical property is reversible or irreversible and/or that the temperature-dependent change in the at least one physical property occurs within not more than 10 seconds, preferably within not more than 2 seconds, after a predefined temperature threshold value has been exceeded.

3. Ophthalmological composition (1) according to Claim 1 or 2,
**characterized in that**
the at least one thermoresponsive compound (3) is selected from a group comprising polymers, interpenetrating polymer networks, semi-interpenetrating polymer networks, liquid crystals, pigments, dyes, inks, microcapsules and any combinations thereof.

4. Ophthalmological composition (1) according to Claim 3,
**characterized in that**
the at least one thermoresponsive compound (3) comprises at least one constitutional unit selected from a group consisting of poly(N-isopropylacrylamide), poly(N,N-diethylacrylamide), polymethyl vinyl ether), poly(N-vinylcaprolactam), a block copolymer of poly(ethylene oxide) and poly(propylene oxide), a poly (pentapeptide) of elastin, an interpenetrating network of polyacrylamide and polyacrylic acid and/or copolymers thereof.

5. Ophthalmological composition (1) according to any of Claims 1 to 4,
**characterized in that**
the at least one viscoelastic polymer (2) comprises a polysaccharide selected from a group consisting of glycosaminoglycans, cellulose, a cellulose ether with methyl and/or ethyl and/or propyl groups, in particular hydroxypropylmethylcellulose, hydroxyethylmethylcellulose and/or methylcellulose, a glycosaminoglycan, in particular hyaluronic acid, chondroitin sulfate, dermatan sulfate, heparin, heparan sulfate, keratan sulfate, alginic acid, polymannuronic acid, polyguluronic acid, polyglucuronic acid, amylose, amylopectin, callose, chitosan, polygalactomannan, dextran, xanthan and/or any mixture thereof, copolymers thereof, and pharmacologically acceptable salts thereof.

6. Ophthalmological composition (1) according to any of Claims 1 to 5,
**characterized in that**
the at least one thermoresponsive compound (3) is covalently linked to the at least one viscoelastic polymer (2), especially via a spacer, and/or **in that** the at least one thermoresponsive compound (3) is present in the form of particles and/or microspheres dispersed in the at least one viscoelastic polymer (2) and/or **in that** the at least one viscoelastic polymer (2) and the at least one thermoresponsive compound (3) form a semi-interpenetrating and/or interpenetrating network.

7. Ophthalmological composition (1) according to any of Claims 1 to 6,
**characterized in that**
the at least one thermoresponsive compound (3) has, in the first temperature range below 60°C, especially below 40°C, and above 35°C, the first colour and/or transmission property and, in the second temperature range that is above the first temperature range, the second colour and/or transmission property that is different from the first colour and/or transmission property.

8. Ophthalmological composition (1) according to Claim 7,
**characterized in that**
the at least one thermoresponsive compound (3) is in the first temperature range at least essentially colourless and/or at least mostly transparent and/or that the at least one thermoresponsive compound (3) is in the second temperature range coloured and/or at least mostly non-transparent.

9. Ophthalmological composition (1) according to any of Claims 1 to 8,
**characterized in that**
the predefined wavelength range is between 50 µm and 200 nm, especially between 780 nm and 315 nm.

## Revendications

1. Composition ophtalmologique (1), comprenant au moins un polymère viscoélastique (2),
**caractérisée en ce que**
celle-ci comprend au moins un composé thermosensible (3) qui, en fonction de la température, modifie de manière discontinue au moins une propriété physique parmi le groupe comprenant la couleur et le degré de transmission dans une plage de longueurs d'onde prédéterminée, ledit au moins un composé thermosensible (3) possédant une première propriété de couleur et/ou de transmission dans une première plage de températures inférieure à 60 °C et une seconde propriété de couleur et/ou de transmission différente de la première propriété de couleur et/ou de transmission dans une seconde plage de températures se situant au-dessus de la première plage de températures.

2. Composition ophtalmologique (1) selon la revendication 1,
**caractérisée en ce que**
une modification thermodépendante de l'au moins une propriété physique est réversible ou irréversible et/ou **en ce que** la modification thermodépendante de l'au moins une propriété physique se produit en au plus 10 secondes, préférablement en au plus 2 secondes, en cas de dépassement d'une valeur limite de température prédéterminée.

3. Composition ophtalmologique (1) selon la revendication 1 ou 2,
**caractérisée en ce que**
l'au moins un composé thermosensible (3) est choisi parmi un groupe qui comprend des polymères, des réseaux de polymère interpénétrants, des réseaux de polymère semi-interpénétrants, des cristaux liquides, des pigments, des colorants, des encres, des microcapsules et n'importe quelle combinaison de ceux-ci.

4. Composition ophtalmologique (1) selon la revendication 3,
**caractérisée en ce que**
l'au moins un composé thermosensible (3) comprend au moins un motif constitutif choisi dans le groupe constitué par un poly(N-isopropylacrylamide), un poly(N,N-diéthylacrylamide), un poly(méthylvinyléther), un poly(N-vinylcaprolactame), un copolymère séquencé de poly(oxyde d'éthylène) et de poly(oxyde de propylène), un poly(pentapeptide) d'élastine, un réseau interpénétrant de polyacrylamide et de poly(acide acrylique), et/ou des copolymères de ceux-ci.

5. Composition ophtalmologique (1) selon l'une quelconque des revendications 1 à 4,
**caractérisée en ce que**
l'au moins un polymère viscoélastique (2) comprend un polysaccharide choisi dans le groupe constitué des glycosaminoglycanes, de la cellulose, d'un éther de cellulose comportant des groupes méthyle et/ou éthyle et/ou propyle, en particulier de l'hydroxypropylméthylcellulose, de l'hydroxyéthylméthylcellulose et/ou de la méthylcellulose, d'un glycosaminoglycane, en particulier de l'acide hyaluronique, du sulfate de chondroïtine, du sulfate de dermatane, de l'héparine, du sulfate d'héparane, du sulfate de kératane, de l'acide alginique, de l'acide polymannuronique, de l'acide polyguluronique, de l'acide polyglucuronique, de l'amylose, de l'amylopectine, de la callose, du chitosane, du polygalactomannane, du dextrane, du xanthane et/ou de n'importe quel mélange de ceux-ci, de leurs copolymères et de leurs sels pharmacologiquement acceptables.

6. Composition ophtalmologique (1) selon l'une quelconque des revendications 1 à 5,
**caractérisée en ce que**
l'au moins un composé thermosensible (3) est lié de manière covalente à l'au moins un polymère viscoélastique (2), en particulier par l'intermédiaire d'un espaceur, et/ou **en ce que** l'au moins un composé thermosensible (3) se présente sous forme de particules et/ou de microsphères réparties dans l'au moins un polymère viscoélastique (2), et/ou **en ce que** l'au moins un polymère viscoélastique (2) et l'au moins un composé thermosensible (3) forment un réseau semi-interpénétré et/ou interpénétré.

7. Composition ophtalmologique (1) selon l'une quelconque des revendications 1 à 6,
**caractérisée en ce que**
l'au moins un composé thermosensible (3) possède, dans la première plage de températures inférieure à 60 °C, en particulier inférieure à 40 °C, et supérieure à 35 °C, la première propriété de couleur et/ou de transmission, et dans la seconde plage de températures située au-dessus de la première plage de températures, la seconde propriété de couleur et/ou de transmission différente de la première propriété de couleur et/ou de transmission.

8. Composition ophtalmologique (1) selon la revendication 7,
**caractérisée en ce que**
l'au moins un composé thermosensible (3) est au moins essentiellement incolore et/ou au moins principalement transparent dans la première plage de températures et/ou l'au moins un composé thermosensible (3) est coloré et/ou au moins principalement opaque dans la seconde plage de températures.

9. Composition ophtalmologique (1) selon l'une quelconque des revendications 1 à 8,
**caractérisée en ce que**
la plage de longueurs d'onde prédéterminée se situe entre 50 µm et 200 nm, en particulier entre 780 nm et 315 nm.
